(19) Europäisches Patentamt
European Patent Office
Office européen des brevets

(11) Publication number: 0 343 367 A1

# (12) EUROPEAN PATENT APPLICATION

(21) Application number: 89106794.4

(22) Date of filing: 17.04.89

(51) Int. Cl.4: C07C 149/00 , C07C 149/26 , A61K 31/10

Claims for the following Contracting States: ES + GR.

(30) Priority: 23.05.88 IT 2070188

(43) Date of publication of application: 29.11.89 Bulletin 89/48

(84) Designated Contracting States: AT BE CH DE ES FR GB GR IT LI LU NL SE

(71) Applicant: CAMILLO CORVI S.p.A.
via S. Marco 18
Milan(IT)

(72) Inventor: Braga Piercarlo
Via Meda 1
I-Rho Milan(IT)

(74) Representative: Klausner, Erich et al
c/o Ufficio Internazionale Brevetti Ing. C. Gregorj S.p.A. Via Dogana 1
I-20123 Milano(IT)

## (54) Mercapto-compounds having mucolytic action and a process for their preparation.

(57) A series of new compounds represented by the following general formula (I)

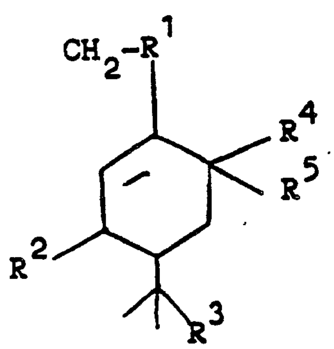

(I)

wherein $R^1$ = H, SH
$R^2$ = H, SH
$R^3$ = OH, SH
$R^4$ = H, OH, SH
$R^5$ = H, OH, SH
and $R^4$ and $R^5$ taken together represent =O is described.
The compounds represented by the general formula (I) possess a mucolytic action.

EP 0 343 367 A1

Xerox Copy Centre

The object of this invention consists of new compounds having mucolytic activity and a process for the preparation thereof. More particularly, the compounds of the invention have the following structural formula :

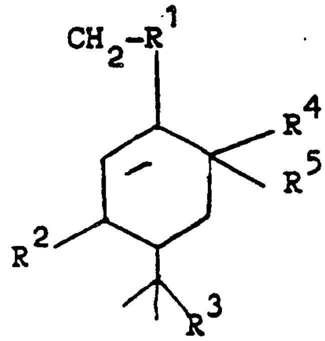

(I)

wherein $R^1$ = H, SH
$R^2$ = H, SH
$R^3$ = OH, SH
$R^4$ = H, OH, SH
$R^5$ = H, OH, SH
and $R^4$ and $R^5$ taken together represent =O.

The new compounds of this invention are prepared according to the following reaction schemes :

Scheme a)

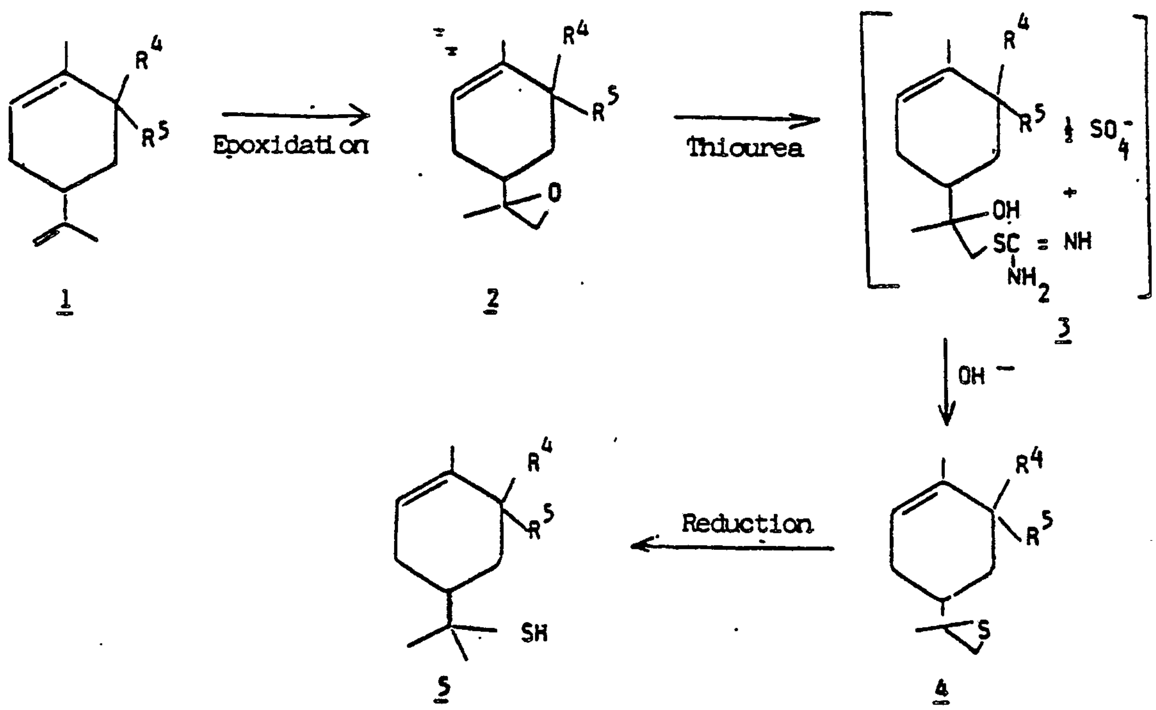


1 2 3 4 5

wherein $R^4$ = H, OH; $R^5$ = H, OH; or $R^4$ and $R^5$ taken together represent =O

Specifically, compound 1 is epoxidized selectively into 2 with m-chloroperbenzoic acid in an anhydrous environment, at low temperature, in the presence of a suitable chlorinated solvent, such as e.g. chloroform . The epoxide 2, diluted in a suitable aprotic - with an alkaline solution, preferably sodium carbonate, thus yielding the episulfide 4 from which -through the reduction with lithium-alluminium hydride in an anhydrous environment, in a suitable solvent such as tetrahydrofuran - the desidered mercaptan 5 is formed.

When the substituents R and R′ of the episulfide 4 represent =0, it will be necessary to protect the

carbonyl group by means of a suitable protecting group. It will be possible to carry out such protection in any step of the procedure that precedes the reduction and, therefore, immediately after reduction has taken place, the protecting group will be removed according to a known technique. A suitable protection consists in transforming the carbonyl group into a ketal and subsequently restoring the carbonyl function by treating with an aqueous solution of hydrogen chloride.

Scheme b)

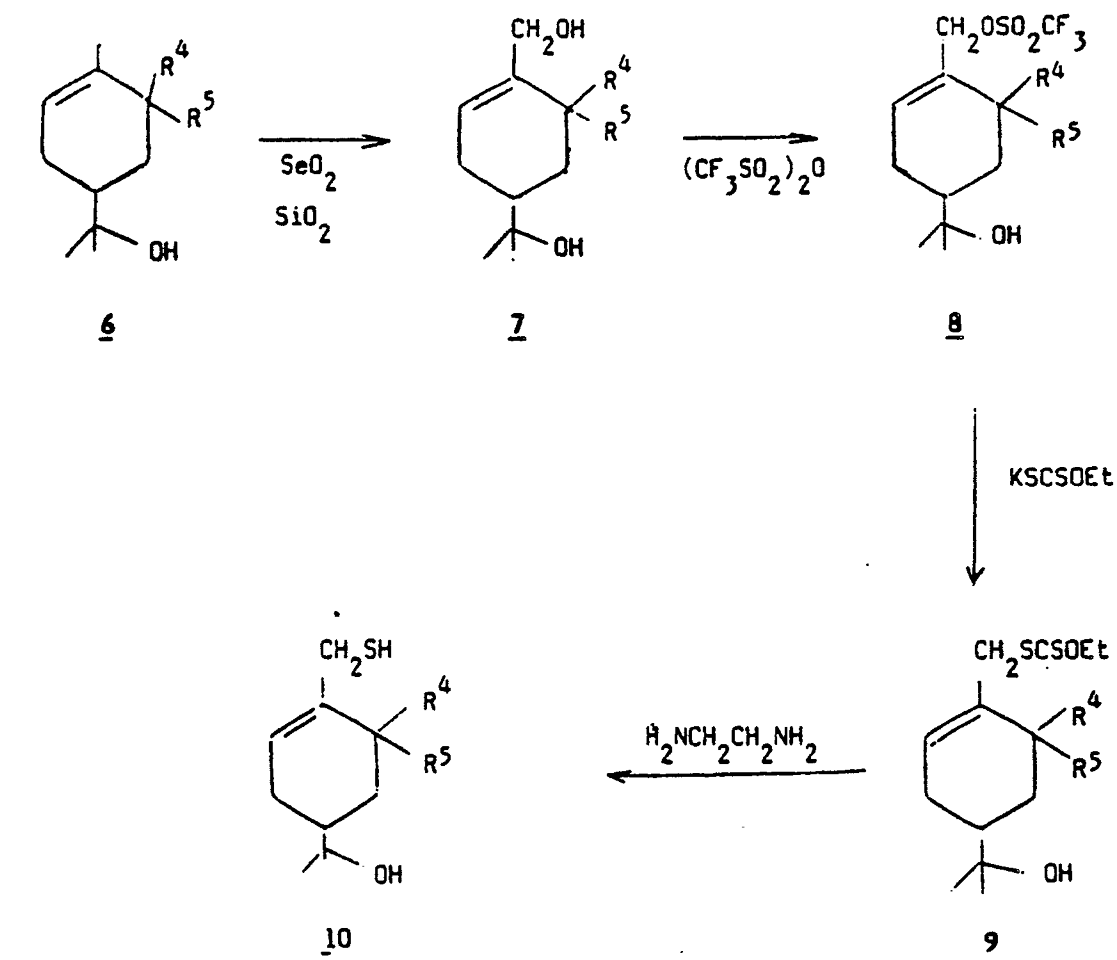


6 7 8 9 10

wherein $R^4$ = H, OH; $R^5$ = H, OH; or $R^4$ and $R^5$ taken together represent =O.

When these substituents represent H, OH, the hydroxyl group is previously suitably protected by means of e.g. a suitable , easily removable acyl group.

Specifically, the starting cyclohexene compound 6 is selectively oxidized by reacting with selenium dioxide adsorbed on silica gel. This reaction is carried out at room temperature in anhydrous conditions in a suitable solvent, preferably chlorinated, such as e.g. dichloromethane or chloroform. The allyl alcohol 7 is then reacted with a suitable acylating agent capable of introducing the trifluoromethane sulfone group, such as e.g. trifluoromethane sulfonic anhydride. This reaction is carried out at low temperature and in anhydrous conditions, at a temperature ranging preferably from 0 to 5 °C, in a suitable solvent such as e.g. an organic base, preferably pyridine.

The triflate 8 is reacted in a suitable aprotic non-polar solvent such as e.g. hexamethylphosphoramide, dimethyl-sulfoxide, formamide and polyethylene glycol, with a suitable salt of ethylxanthic acid, and the thus obtained corresponding ethylxanthogenate 9, by reaction with ethylenediamine in tetrahydrofuran, yields the desired mercaptan 10.

Scheme c)

When, in the first step of the above-mentioned procedure (Scheme b), the oxidation reaction is carried out with selenium dioxide but without silica, the intro duction of a hydroxyl group at the position 2 of the cyclohexene ring occurs : this hydroxyl group is treated as described in Scheme b), i.e.

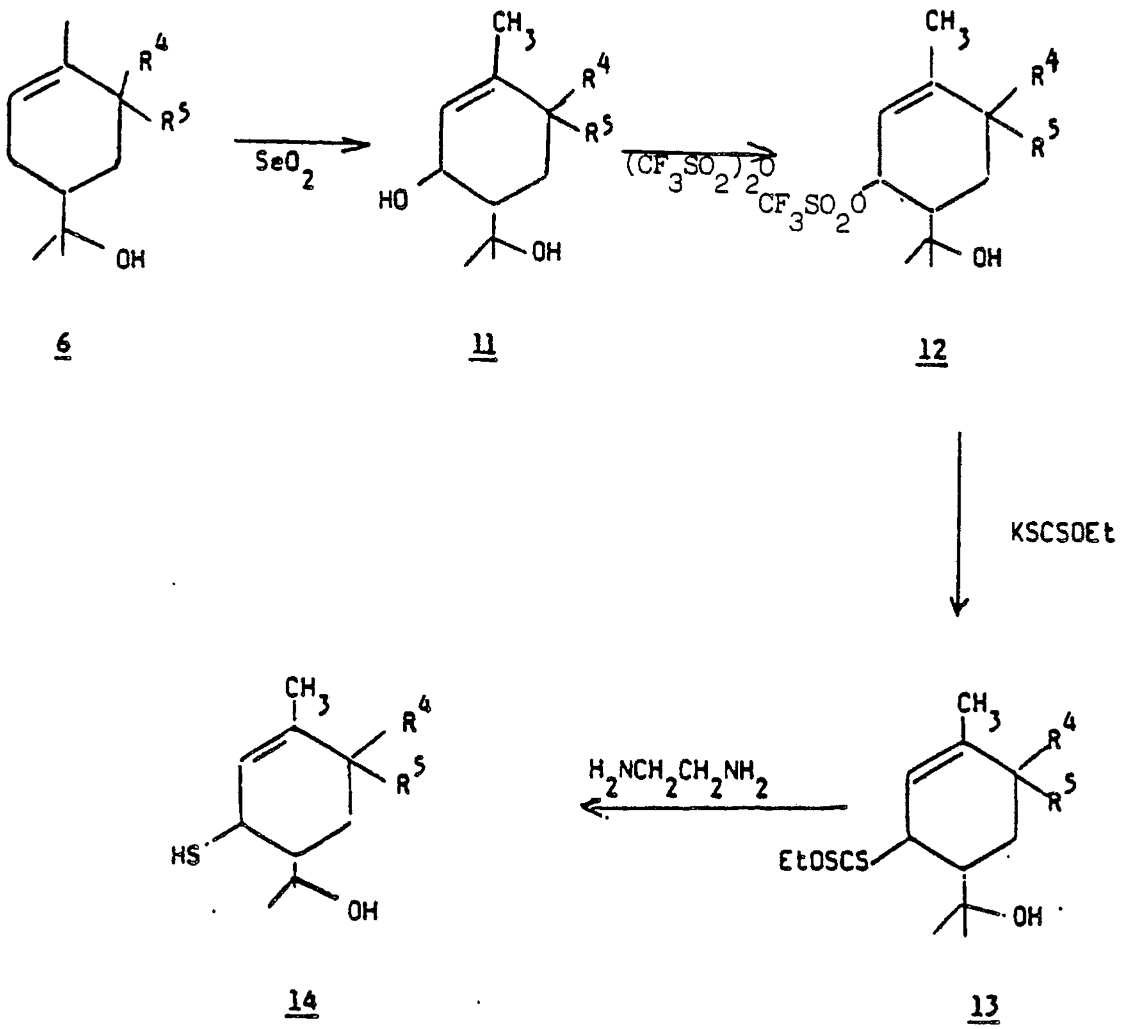


6 11 12

14 13

wherein $R^4$ = H, OH; $R^5$ = H, OH; or $R^4$ and $R^5$ taken together represent =O.

When these substituents represent H, OH, the hydroxyl group is previously appropriately protected e.g. by means of a suitable, easily removable acyl group.

Scheme d)

When, in the starting compound 6, $R^4$ and $R^5$ represent H, OH and the hydroxyl group is not suitably protected, the introduction of the mercapto-group occurs by directly treating the compound 6 with a suitable acylating agent capable of introducing the trifluoromethane sulfone group, then wth a suitable salt of ethylxanthic acid and finally with ethylenediamine according to the handling methods followed in the corresponding above-mentioned steps, via compounds 15 and 16, to yield the mercaptan 17.

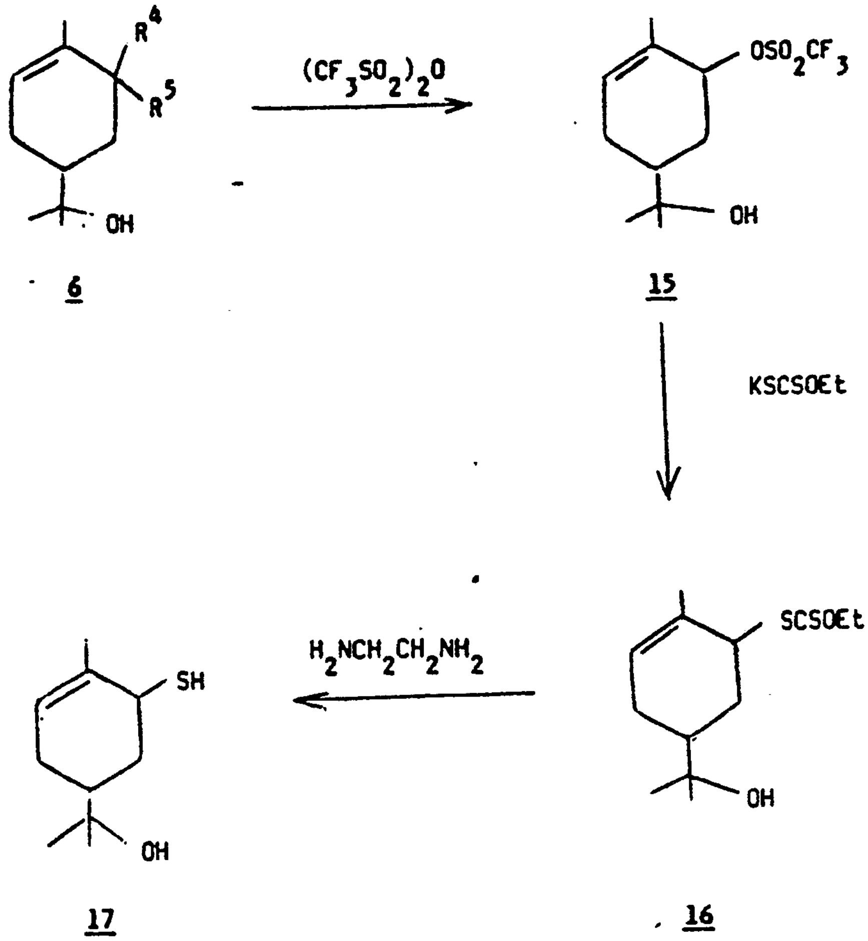


6 15 16 17

The following examples serve the purpose of illustrating the invention, though without limiting it.

## Example 1

### 5-[1-mercapto-1-methylethyl]-2-methyl-2-cyclohexene-1-one

First step:

3 g Carvone[2-methyl-5-(1-methylethenyl)-2-cyclohexene-1-one] (1, $R^4$ and $R^5$ taken together =0), in 50 ml anhydrous dichloromethane is treated with 3.4 g m-chloroperbenzoic acid and the mixture is left standing for 2 hours in an external ice bath. After washing with sodium bicarbonate and anhydrating on sodium sulfate, the mixture is evaporated to dryness and 1.8 g carvone epoxide is obtained (2, $R^4$ and $R^5$ taken together =0) having a boiling point of 94 - 96 °C (14 mm Hg).

Second step:

1.8 g Carvone epoxide (2, $R^4$ and $R^5$ taken together =0) in 5 ml tetrahydrofuran is added with a solution containing 1.36 g thiourea, 0.46 ml concentrated sulfuric acid and 12 ml water; the reaction mixture is reacted for 10 minutes at 5 °C and then for 18 hours at 220 °C, and then filtered; the filtrate is treated with aqueous sodium carbonate, leaving the reaction mixture at 20 °C for 20 minutes and at 50 °C for 15 minutes.

After extraction with dichloromethane 2 g crude carvone episulfide is obtained which, upon purification

on a silica gel column (eluant: petroleum ether/ethyl acetate, 7:3) yields 700 mg pure carvone episulfide (4, $R^4$ and $R^5$ taken together =0) having a boiling point of 98-100° C (16 mm Hg).

Third step:

450 mg Carvone episulfide (4, $R^4$ and $R^5$ taken together =0) is reacted 2 hours with 365 mg lithium-alluminium hydryde in 10 ml anhydrous tetrahydrofuran; after adding 1N sodium hydroxide, 300 mg 5 [1-mercapto-1-methylethyl]-2-methyl-2-cyclohexene-1-one (5, $R^4$ and $R^5$ taken together =0) is obtained; b.p.: 146-148° C (1 mm Hg).

Example 2

5-hydroxy-α,α,4-trimethyl-3-cyclohexen-1-methanethiol

Following a similar procedure to that previously described, using as starting material 2-methyl-5-(1-methylethenyl)-2-cyclohexene-1-ol (1, $R^4$ =H, $R^5$ =OH), 5-hydroxy-α,α,4-trimethyl-3-cyclohexene-1-methylmercaptan (5, $R^4$ =H, $R^5$ =OH) is obtained.

Example 3

2-hydroxy-4-(1-hydroxy-1-methylethyl)-6-cyclohexene-1-methanethiol

First step:

1 g Sobrerol (6, $R^4$ =H, $R^5$ =OH) is reacted for 16 hours at room temperature with 5 ml acetic anhydryde and 5 ml pyridine.

Upon pouring the solution into water, it is extracted three times with 5 ml dichloromethane and the solvent is removed by evaporation. The residue, upon having been taken up several times with toluene, yields 1.1 g sobrerol acetate; b.p.:130-132° C (14 mm Hg).

1 g Sobrerol acetate is added with an $SeO_2/SiO_2$ suspension at 5% $SeO_2$, treated with t-buthyl-hydroperoxide in 25 ml dichloromethane.

The reaction mixture is mixed for 3 hours at room temperature; the solid is then separated by filtration and the organic solution is evaporated to dryness yielding 700 mg 6-acetiloxy-$\alpha^4$,$\alpha^4$-dimethyl-1-cyclohexene-1,4-dimethanol (7, $R^4$ =H, $R^5$ =OAc) which, upon recrystallization from methyl alcohol/isopropyl ether, melts at 155-157° C.

Second step:

700 mg 6-Acetyloxy-$\alpha^4$,$\alpha^4$-dimethyl-1-cyclohexene-1,4-dimethanol is dissolved at low temperature in 10 ml pyridine and the solution is then added with a solution of 0.7 ml trifluoromethane sulfonic anhydride in 5 ml pyridine at 3° C.

The solution is heated to room temperature and left standing for 1 hour, then it is extracted with cold dichloromethane and treated with a 1M solution of sulfuric acid.

0.9 mg of the corresponding trifluoromethansulfonate is obtained which, upon solution in 5 ml anhydrous hexamethylphosphoramide, is treated with 0.5 g potassium ethylxanthogenate.

0,9 mg of the corresponding ethylxanthogenate is thus isolated (9, $R^4$ =H, $R^5$ =OAc), and purified on a silica gel column (eluant: petroleum ether/ethyl acetate, 7:3); m.p.: 145-146° C.

Third step:

700 mg Ethylxanthogenate in 5 ml anhydrous tetrahydrofuran is treated for two hours at room temperature with 0.5 g of ethylenediamine. The solution is then treated with 1M sulfuric acid, extracted with diethyl ether, the solvent is then removed by evaporation and the residue is treated with 1 ml of an ethanol solution of 2M potassium hydroxide. The compound is acidified with 1N hydrochloric acid and 400 mg 2-hydroxy-4-(1-hydroxy-1-methylethyl)-6-cyclohexene-1-mercaptomethyl (10, $R^4 = H$, $R^5 = OH$) is obtained as a precipitate which, upon recrystallization from ethyl alcohol/isopropyl ether, melts at 138-139 °C.

Example 4

5-(1-hydroxy-1-methylethyl)-2-mercaptomethyl-2-cyclohexene-1-one

The procedure is the same as the one previously described starting from carvone hydrate (6, $R^4$ and $R^5$ taken together = 0) instead of sobrerol acetate whereupon 5-(1-hydroxy-1-methylethyl)-2-mercaptomethyl-2-cyclohexene-1-(10, $R^4$ and $R^5$ taken together = 0)is yielded.

Example 5

5-hydroxy-2-mercapto-α,α,4-trimethyl-3-cyclohexene-1-methanol

First step:

1.2 g $SeO_2$ is added with 1 g sobrerol acetate (6, $R^4 = H$, $R^5 = OAc$), prepared according to Example 3, in 10 ml dioxane. The mixture is refluxed for 3 hours and after separating the solid by filtration, the organic solution is evaporated to dryness yielding 650 mg 5-acetiloxy-2-hydroxy-α,α,4-trimetil-3-cyclohexene-1-methanol which is purified on a silica gel column (eluant: petroleum ether/ethyl ether 7:3) and recrystallized from ethyl alcohol/isopropyl ether; m.p.: 166-167 °C.

Second step:

420 mg 5-Acetyloxy-2-hydroxy-α,α,4-trimethyl-3- cyclohexene-1-methanol is dissolved at low temperature in 2 ml pyridine and added with a solution of 0.5 ml trifluoromethane sulfonic anhydride in 2 ml pyridine at 3 °C.

The solution is heated to room temperature and left standing for 1 hour. It is then extracted with cold dichloromethane, treated with a solution of 1M sulfuric acid yielding 0.9 g of the corresponding triflate which is dissolved in 5 ml anhydrous methylfosforamide and thereafter reacted with 0.5 g potassium ethylxanthogenate.

0.6 g of the corresponding ethylxanthogenate is isolated (13, $R^4 = H$, $R^5 = OAc$) which is then purified on a silica gel column (eluant: petroleum ether/ethyl acetate, 8:2), recrystallized from isopropyl ether; m.p.: 138-140 °C.

Third step:

200 mg Ethylxanthogenate in 2 ml of tetrahydrofuran is reacted for 2 hours, at room temperature with 0.3 g ethylendiamine.

The solution is then treated with a solution of 1M sulfuric acid; extracted with diethyl ether, whereupon the solvent is removed by evaporation and the residue is treated with an ethanol solution of potassium hydroxide. Upon hydrochloric acidification with 1N acid, 400 mg 5- hydroxy-2-mercapto-α,α,4-trimethyl-3-cyclohexene-1-methanol, recrystallized from ethyl alcohol/isopropyl ether is obtained; m.p.: 128-130 °C.

Example 6

5-(1-hydroxy-1-methylethyl)-2-methyl-4-mercapto-2-cyclohexene-1-one

The procedure is the same as described in Example 5, starting from carvone hydrate (6, $R^4$ and $R^5$ together =0) instead of sobrerol acetate: 5-(1-hydroxy-1-methylethyl)-2-methyl-4-mercapto-2-cyclohexene-1-one (14, $R^4$ and $R^5$ taken together =0) is obtained.

Example 7

5-mercapto-α,α,4-trimethyl-3-cyclohexene-1-methanol

First step:

2 g Sobrerol (6, $R^4$ =H, $R^5$ =OH) is dissolved in 10 ml pyridine at low temperature and this solution is added with a solution consisting of 1 ml trifluoromethane sulfonic anhydride in 5 ml pyridine at 3°C.

The solution is then heated to room temperature and left standing for 1 hour.

It is extracted with cold dichloromethane and treated with a solution of 1M sulfuric acid.

1.8 g of the corresponding triflate (15) is obtained which, upon dissolution in 5 ml anhydrous examethylfosforamide, is reacted with 2 g potassium ethylxanthogenate.

When the reaction is over, 1.1 g of the corresponding ethylxanthogenate (16) is isolated and purified on a silica gel column (eluant: petroleum ether/ethyl acetate: 7:3), recrystallized from isopropanol/isopropyl ether; m.p.: 122-124°C.

Second step:

700 mg of the so obtained ethylxanthogenate (16) in 5 ml anhydrous tetrahydrofuran is reacted for two hours with 0.5 g ethylendiamine.

The solution is then reacted with a 1M sulfuric acid solution and extracted with diethyl ether. The solvent is removed by evaporation and the residue is treated with 1 ml 2M potassium hydroxide in ethanol; the solution is acidified with 1N hydrochloric acid yielding 400 g 5-mercapto-α,α,4-trimethyl-3-cyclohexene-1-methanol (17) which is recrystallized from isopropyl ether; m.p.: 118-120°C.

The compounds of formula(I) have proved to possess a marked mucolytic acitivity which is also effective via inhalation and which makes them thus suitable in all those cases in which there is reactive hyperproduction of bronchial mucus. In these cases, aerosol administration is preferred to oral administration.

The following table indicates e.g. the bronchial secretion stimulating and mucolytic activity of the compound 10 ($R^4$ =H, $R^5$ =OH) as compared to sobrerol.

| Production of mucus in the healthy rabbit [1] Administration route : oral | | | Expectorating activity in the rat[2] | "In vitro" viscometry[3] |
|---|---|---|---|---|
| | Production of mucus | No.of animals/increase | | |
| Sobrerol<br>Compound 10 | $ED_{50}$ 500 mg/kg<br>$ED_{50}$ 400 mg/kg | $ED_{50}$ 500 mg/kg<br>$ED_{50}$ 400 mg/kg | $ED_{50}$ os 40 mg/kg<br>$ED_{50}$ os 100 mg/kg | 2% = + 0,7 %<br>2% = - 5 %<br>10% = - 12 % |

1) According to R. Scuri et al.; Boll. Chim.Farm. 119, 181, 1980

2) According to K. Kogi et al .; Arzneim.Forsch./Drug Research 33, 1281, 1983

3) According to S.S, Davis et al.; Europ. J. Resp. Dis., 67, 94, 1785.

Besides, the acute toxicity of the compounds that are the object of this invention has been tested in mice using the method devised by J. T. Lichtfield and F. Wilcoxon; J.Pharmacol. 96, 99, 1949. The doses administered were the following: 200, 500 and 1000 mg/kg/os. Neither mortality nor toxic symptomatology ascribable to the treatment have been noticed, not even at very high doses.

A further object of this invention are the pharmaceutical preparations having a mucolytic activity that contain the compounds of formula(I) and their derivatives in an active quantity, appropriately mixed with a suitable, pharmaceutically acceptable diluent.

## Claims

1. Pharmacologically active mercapto-compounds having the following structural formula:

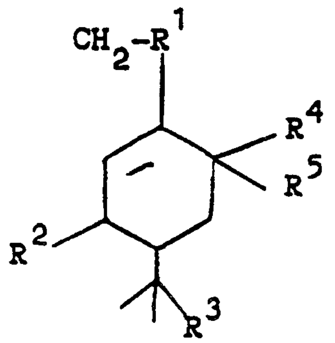

(I)

wherein $R^1$ = H, SH
$R^2$ = H, SH
$R^3$ = OH, SH
$R^4$ = H, OH, SH
$R^5$ = H, OH, SH
and $R^4$ and $R^5$ taken together represent =O ,
with the proviso that when :
$R^1$ = SH, then : $R^2$ represents H; $R^3$ represents OH ; $R^4$ and $R^5$ represent H, OH, or, taken together, they represent =O;
$R^2$ = SH, then : $R^1$ represents H; $R^3$ represents OH ; $R^4$ and $R^5$ represent H, OH, or, taken together, they represent =O;
$R^3$ = SH, then: $R^1$ and $R^2$ represent H; $R^4$ and $R^5$ represent H, OH, or, taken together, they represent =O,
$R^4$ and $R^5$ represent H, SH then: $R^1$ and $R^2$ represent H, and $R^3$ represent OH.

2. A process for making a pharmacologically active compound of formula (I) in claim 1, characterized in that the menthadiene (1) is selectively epoxidized with m-chloroperbenzoic acid, in an anhydrous environment , at low temperature , in the presence of a suitable chlorinated solvent, and the thus obtained epoxide (2), diluted in a suitable aprotic solvent, is then reacted with thirourea and concentrated sulfuric acid, yielding the corresponding thiouronium salt (3) which is then directly treated with an alkaline solution to yield the episulfide (4) which is in turn subjected to reduction with lithium-aluminium hydride, in an anydrous environment and in a suitable solvent.

3. A process according to claim 2, characterized in that when the substituents R and $R^1$, taken together, in the episulfide of formula (4) represent =O , the carbonyl group is protected prior to the reduction step, by means of a removable protecting group.

4. A process according to claim 2, characterized in that when a cyclohexene of formula (6) is used as the starting compound, said cyclohexene is selectively oxidized using selenium dioxide adsorbed on silica gel, at room temperature and in anhydrous conditions, in a suitable chlorinated solvent, then the thus obtained allyl alcohol (7) is reacted at a temperature of from 0 to 5 °C, and in a solvent consisting of an organic base, with a suitable acylating agent capable of introducing the trifluoromethane sulfone group, and thereafter the so obtained triflate (8) is reacted in a suitable aprotic non-polar solvent, with an ethylxanthate to yield the corresponding ethylxanthogenate (9), which in turn is reacted with ethylenediamine in tetrahydrofuran.

5. A process according to claim 4, characterized in that the oxidation reaction is carried out using unadsorbed selenium dioxide, whereby the hydroxyl group, at the position 2 of the thus obtained hydroxyl derivative (11), has to be adequately protected by means of a suitable, easily removable, acyl group when the substituents $R^4$ and $R^5$ represent H or OH.

6. A process according to claim 4, characterized in that when in the cyclohexene of formula (6) the substituents $R^4$ and $R^5$ represent H or OH, and the hydroxyl group is not protected, the mercapto group is introduced directly by reacting said cyclohexene with a suitable acylating agent.

7. A process for making a pharmaceutical preparation having mucolytic activity, characterized in that a therapeutically active quantity of a compound according to claim 1 is admixed with a suitable pharmaceutically acceptable diluent.

Claims for the following Contracting State: ES

1. A process for making pharmacologically active mercapto-compound having the following structural formula:

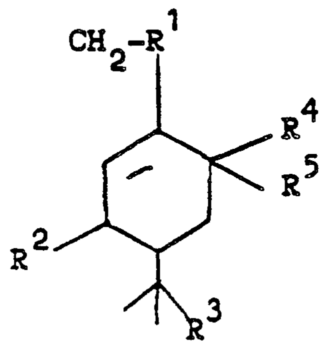

(I)

wherein $R^1$ = H, SH
$R^2$ = H, SH
$R^3$ = OH, SH
$R^4$ = H, OH, SH
$R^5$ = H, OH, SH
and $R^4$ and $R^5$ taken together represent =O ,
with the proviso that when :
$R^1$ = SH, then : $R^2$ represents H; $R^3$ represents OH ; $R^4$ and $R^5$ represent H, OH, or, taken together, they represent =O;
$R^2$ = SH, then : $R^1$ represents H; $R^3$ represents OH ; $R^4$ and $R^5$ represent H, OH, or, taken together, they represent =O;
$R^3$ = SH, then: $R^1$ and $R^2$ represent H; $R^4$ and $R^5$ represent H, OH, or taken together, they represent =O,
$R^4$ and $R^5$ represent H, SH,then: $R^1$ and $R^2$ represent H, and $R^3$ reprents OH,
characterized in that the menthadiene (1) is selectively epoxidized with m-chloroperbenzoic acid, in an anhydrous envirnment, at low temperature, in the presence of a suitable chlorinated solvent, and the thus obtained epoxide (2), diluted in a suitable aprotic solvent, is then reacted with thiourea and concentrated sulfuric acid, yielding the corresponding thiouronium salt (3) which is then directly treated with an alkaline solution to yield the episulfide (4) which is in turn subjected to reduction with lithium-aluminium hydride,in an anhydrous environment and in a suitable solvent.

2. A process according to claim 1, characterized in that when the substituents R and $R^1$ , taken together, in the episulfide of formula (4) represent =O , the carbonyl group is protected prior to the reduction step, by means of a removable protecting group.

3. A process according to claim 1, characterized in that when a cyclohexene of formula (6) is used as the starting compound, said cyclohexene is selectively oxidized using selenium dioxide adsorbed on silica gel, at room temperature and in anhydrous conditions, in a suitable chlorinated solvent , then the thus obtained allyl alcohol (7) is reacted at a temperature of from 0 to 5° C, and in a solvent consisting of an organic base, with a suitable acylating agent capable of introducing the trifluoromethane sulfone group, and thereafter the so obtained triflate (8) is reacted in a suitable aprotic non-polar solvent, with an ethylxanthate to yield the corresponding ethylxanthogenate (9), which in turn is reacted with ethylenediamine in tetrahydrofuran.

4. A process according to claim 4, characterized in that the oxidation reaction is carried out using unadsorbed selenium dioxide, whereby the hydroxyl group, at the position 2 of the thus obtained hydroxyl derivative (11), has to be adequately protected by means of a suitable, easily removable, acyl group when the substituents $R^4$ and $R^5$ represent H or OH.

5. A process according to claim 3, characterized in that when in the cyclohexane of formula (6) the substituents $R^4$ and $R^5$ represent H or OH, and the hydroxyl group is not protected, the mercapto group is introduced directly by reacting said cyclohexene with a suitable acylating agent.

6. A process for making a pharmaceutical preparation having mucolytic activity, characterized in that a therapeutically active quantity of a compound according to claim 1 is admixed with a suitable pharmaceutically acceptable diluent.

Claims for the following Contracting State: GR

1. Pharmacologically active mercapto-compounds having the following structural formula:

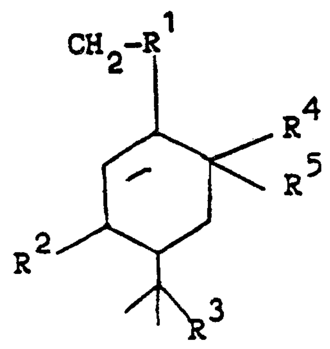

(I)

wherein $R^1$ = H, SH
$R^2$ = H, SH
$R^3$ = OH, SH
$R^4$ = H, OH, SH
$R^5$ = H, OH, SH
and $R^4$ and $R^5$ taken together represent =O ,
with the proviso that when :
$R^1$ = SH, then : $R^2$ represents H; $R^3$ represents OH ; $R^4$ and $R^5$ represent H, OH, or, taken together, they represent =O ;
$R^2$ = SH, then : $R^1$ represents H; $R^3$ represents OH ; $R^4$ and $R^5$ represent H, OH, or, taken together, they represent =O;
$R^3$ = SH, then: $R^1$ and $R^2$ represent H;$R^4$ and $R^5$ represent H,OH or, taken together, they represent =O;
$R^4$ and $R^5$ represent H, SH,then : $R^1$ and $R^2$ represent H, and $R^3$ represent OH.

2. A process for making a pharmacologically active compound of formula (I) in claim 1, characterized in that the menthadiene(1) is selectively epoxidized with m-chloroperbenzoic acid, in an anhydrous environment, at low temperature, in the presence of a suitable chlorinated solvent, and the thus obtained epoxide (2), diluted in a suitable aprotic solvent, is then reacted with thiourea and concentrated sulfuric acid, yielding the corresponding thiouronium salt (3) which is then directly treated with an alkaline solution to yield the episulfide (4) which is in turn subjected to reduction with lithium-aluminium hydride, in an anhydrous environment and in a suitable solvent.

3. A process according to claim 2, characterized in that when the substituents R and $R^1$, taken together, in the episulfide of formula (4) represent =O , the carbonyl group is protected prior to the reduction step, by means of a removable proctecting group.

4. A process according to claim 2, characterized in that when a cyclohexene of formula (6) is used as the starting compound, said cyclohexene is selectively oxidized using selenium dioxide adsorbed on silica gel, at room temperature and in anhydrous conditions, in a suitable chlorinated solvent, then the thus obtained allyl alcohol (7) is reacted at a temperature of from 0 to 5° C, and in a solvent consisting of an organic base, with a suitable acylating agent capable of introducing the trifluoromethane sulfone group, and

thereafter the so obtained triflate (8) is reacted in a suitable aprotic non-polar solvent, with an ethylxanthate to yield the corresponding ethylxanthogenate (9), which in turn is reacted with ethylenediamine in tetrahydrofuran.

5. A process according to claim 4, characterized in that the oxidation reaction is carried out using unadsorbed selenium dioxide, whereby the hydroxyl group, at the position 2 of the thus obtained hydroxyl derivative (11), has to be adequately protected by means of a suitable, easily removable, acyl group when the substituents $R^4$ and $R^5$ represent H or OH.

6. A process according to claim 4, characterized in that when in the cyclohexene of formula (6) the substituents $R^4$ and $R^5$ represent H or OH, and the hydroxyl group is not protected , the mercapto group is introduced directly by reacting said cyclohexene with a suitable acylating agent.

7. A pharmaceutical preparation having a mucolytic activity containing a therapeutically active quantity of a compound according to claim 1, in admixture with a suitable, pharmaceutically acceptable diluent.

European Patent Office

# EUROPEAN SEARCH REPORT

Application Number

EP 89 10 6794

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| | No relevant documents have been disclosed<br>----- | | C 07 C 149/00<br>C 07 C 149/26<br>A 61 K 31/10 |
| | | | **TECHNICAL FIELDS SEARCHED (Int. Cl.4)** |
| | | | C 07 C 149/00 |

The present search report has been drawn up for all claims

| Place of search | Date of completion of the search | Examiner |
|---|---|---|
| THE HAGUE | 26-07-1989 | ZAROKOSTAS K. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)